(19) Europäisches Patentamt
European Patent Office
Office européen des brevets

(11) Veröffentlichungsnummer: **0 008 080**
**B1**

(12) # EUROPÄISCHE PATENTSCHRIFT

(45) Veröffentlichungstag der Patentschrift: **01.07.81**

(51) Int. Cl.[3]: **A 61 K 7/13**

(21) Anmeldenummer: **79102773.3**

(22) Anmeldetag: **02.08.79**

(54) **Mittel zur Färbung von Haaren.**

(30) Priorität: **16.08.78 DE 2835776**

(43) Veröffentlichungstag der Anmeldung: **20.02.80 Patentblatt 80/4**

(45) Bekanntmachung des Hinweises auf die Patenterteilung: **01.07.81 Patentblatt 81/26**

(84) Benannte Vertragsstaaten: **AT IT**

(56) Entgegenhaltungen:
**EP-A-0 004 366**
**US-A-2 391 137**

(73) Patentinhaber: **Wella Aktiengesellschaft, Berliner Allee 65, D-6100 Darmstadt (DE)**

(72) Erfinder: **Konrad, Eugen, Mecklenburger Strasse 101, D-6100 Darmstadt (DE)**
Erfinder: **Mager, Herbert, Dr., Beaumont 5, CH-1700 Fribourg (CH)**

**EP 0 008 080 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Mittel zur Färbung von Haaren

Gegenstand der Erfindung sind Mittel zur oxidativen Färbung von Haaren, die gekennzeichnet sind durch einen Gehalt an Derivaten des 1,2-Methylendioxy-benzols als Kupplersubstanz.

Oxidationsfarbstoffe haben auf dem Gebiet der Haarfärbung eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels.

Als Entwicklersubstanzen werden insbesondere 1,4-Diaminobenzol, 2,5-Diaminotoluol und p-Aminophenol eingesetzt. Von den vorzugsweise verwendeten Kupplersubstanzen sind Resorcin, 4-Chlorresorcin, $\alpha$-Naphthol, m-Aminophenol und Derivate des m-Phenylendiamins zu nennen.

An Oxidationsfarbstoffe, die zur Färbung menschlicher Haare Verwendung finden, sind zahlreiche besondere Anforderungen gestellt. So müssen sie in toxikologischer und dermatologischer Hinsicht unbedenklich sein und Färbungen in der gewünschten Intensität ermöglichen. Außerdem ist es erforderlich, daß durch Kombination geeigneter Entwickler- und Kupplerkomponenten eine breite Palette verschiedener Farbnuancen erzeugt werden kann. Ferner wird für die erzielbaren Haarfärbungen eine gute Licht-, Dauerwell-, Säure- und Reibechtheit gefordert. Auf jeden Fall aber müssen solche Haarfärbungen ohne Einwirkung von Licht, Reibung und chemischen Mitteln über einen Zeitraum von mindestens 4 bis 6 Wochen stabil bleiben.

Der Vielzahl der gestellten Anforderungen können die zur Zeit in Oxidationshaarfärbemitteln verwendeten sowie auch die in neuerer Zeit empfohlenen Farbstoffvorstufen wie beispielsweise Pyrimidinderivate jedoch nicht völlig zufriedenstellend genügen.

In dem Bestreben, gegenüber Mitteln zur oxidativen Färbung von Haaren auf der Basis der Entwicklersubstanzen 1,4-Diaminobenzol und 2,5-Diaminotoluol in toxikologischer und dermatologischer Hinsicht eine Verbesserung zu erreichen, wird heute versucht, diese Entwicklersubstanzen zunehmend durch das physiologisch verträglichere p-Aminophenol zu ersetzen. Mit Haarfärbemitteln auf der Basis von p-Aminophenol in Kombination mit bekannten Kupplern lassen sich jedoch weder natürliche Farbtöne erzeugen, noch wird eine ausreichende Farbintensität erreicht. So war bisher der Ersatz von 1,4-Diaminobenzol bzw. von 2,5-Diaminotoluol durch p-Aminophenol nur in Ausnahmefällen möglich.

Es bestand daher die Aufgabe, geeignete Kupplersubstanzen zu finden, die in Kombination mit der physiologisch vorteilhaften Entwicklersubstanz p-Aminophenol den Anforderungen bezüglich Natürlichkeit und Intensität der erzielbaren Färbungen, unter Erhalt der physiologischen Vorteile, genügen.

Hierzu wurde nun gefunden, daß Mittel zur oxidativen Färbung von Haaren mit einem Gehalt an mindestens einem Derivat des 1,2-Methylendioxybenzols der allgemeinen Formel

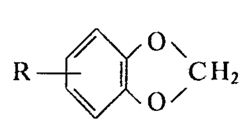

in der R eine OH-, $NH_2$-, $NHR^1$- oder $NR^1R^2$-Gruppe bedeutet, wobei $R^1$ und $R^2$ unabhängig voneinander einen Alkyl-, Hydroxyalkyl- oder Acylrest mit 1 bis 4 Kohlenstoffatomen darstellen, als Kupplersubstanz den gestellten Anforderungen in besonders hohem Maße genügen.

Wenn auch die Verwendung der vorstehenden Derivate des 1,2-Methylendioxy-benzols als Kupplersubstanz in Kombination mit der Entwicklersubstanz p-Aminophenol in physiologischer Hinsicht besonders vorteilhaft ist, so können diese Kupplersubstanzen in den erfindungsgemäßen Haarfärbemitteln, je nach den Erfordernissen, auch in Kombination mit den Entwicklersubstanzen 1,4-Diaminobenzol, 2,5-Diaminotoluol und weiteren bekannten Entwicklersubstanzen oder Gemischen davon enthalten sein.

Die als Kupplersubstanz enthaltenen Derivate des 1,2-Methylendioxy-benzols der angegebenen Formel sind gut in Wasser bzw. Wasser-Alkohol löslich und weisen eine ausgezeichnete Lagerbeständigkeit, insbesondere als Bestandteil der erfindungsgemäßen Haarfärbemittel, auf.

In den Haarfärbemitteln sollen die genannten Kupplersubstanzen, von denen das 4-Hydroxy-1,2-methylendioxy-benzol (auch unter der Trivialbezeichnung »Sesamol« bekannt) bevorzugt wird, in einer Konzentration von etwa 0,01 bis 3,0 Gewichtsprozent, vorzugsweise 0,1 bis 3,0 Gewichtsprozent, enthalten sein. Die Gesamtmenge der Oxidationsfarbstoffe, bestehend aus den Entwicklersubstanzen und den Kupplersubstanzen, beträgt zweckmäßigerweise etwa 0,1 bis 5,0 Gewichtsprozent, vorzugsweise 0,5 bis 3,0 Gewichtsprozent.

Die Kupplerkomponenten werden im allgemeinen in etwa äquimolaren Mengen, bezogen auf die Entwicklerkomponenten, eingesetzt. Es ist jedoch nicht nachteilig, wenn die Kupplerkomponente diesbezüglich in einem gewissen Unter- oder Überschuß vorhanden ist.

Darüber hinaus können die Haarfärbemittel der vorliegenden Anmeldung zusätzlich andere bekannte Kupplersubstanzen wie $\alpha$-Naphthol, m-Aminophenol, m-Phenylendiamin, m-Toluylendiamin, 2,4-Diaminoanisol, 3,4-Diaminobenzoesäure und 6-Methyl-3-amino-phenol sowie ferner übliche direktziehende Farbstoffe, beispielsweise Triphenylmethanfarbstoffe wie Diamond Fuchsin (C.I. 42 510) und Leather Ruby HF (C.I. 42 520), aromatische Nitrofarbstoffe wie 2-Nitro-1,4-diaminobenzol, Azofarbstoffe wie Acid Brown 4 (C.I. 14 805) und Acid Blue 135 (C.I. 13 385), Anthrachinonfarbstoffe wie Disperse Violet 4 (C.I. 61 105), Disperse Blue 1 (C.I. 64 500), Disperse Red 15 (C.I. 60 710), Disperse Violet 1 (C.I. 61 100), außerdem 1,4,5,8-Tetraamino-anthrachinon und 1,4-Diamino-anthrachinon enthalten.

Außerdem können in den Haarfärbemitteln noch weitere übliche kosmetische Zusätze, beispielsweise Antioxidantien wie Ascorbinsäure oder Natriumsulfit, Alkalihydroxide, Parfümöle, Komplexbildner, Netzmittel, Emulgatoren, Verdicker, Pflegestoffe und andere enthalten sein.

Die Zubereitungsform kann aus einer Lösung, vorzugsweise aus einer Creme, einem Gel oder einer Emulsion, bestehen. Ihre Zusammensetzung stellt eine Mischung der Farbstoffkomponenten mit den für solche Zubereitungen üblichen Bestandteilen dar. Als übliche Bestandteile von Cremes, Emulsionen oder Gelen kommen beispielsweise Netzmittel oder Emulgatoren aus den Klassen der anionischen oder nichtionogenen oberflächenaktiven Substanzen wie Fettalkoholsulfate, Fettsäurealkanolamide, Alkylsulfonate, Alkylbenzolsulfonate, oxäthylierte Fettalkohole, oxäthylierte Nonylphenole, ferner Verdicker wie höhere Fettalkohole, Stärke, Cellulosederivate, Paraffinöl und Fettsäuren sowie außerdem Pflegestoffe wie Lanolinderivate, Cholesterin und Pantothensäure in Betracht.

Die erwähnten Bestandteile werden in den für solche Zwecke üblichen Mengen verwendet, z. B. die Netzmittel und Emulgatoren in Konzentrationen von etwa 0,5 bis 30 Gewichtsprozent, während die Verdicker in einer Menge von etwa 0,1 bis 25 Gewichtsprozent in den Zubereitungen enthalten sein können.

Je nach Zusammensetzung können die erfindungsgemäßen Färbemittel schwach sauer, neutral oder alkalisch reagieren. Insbesondere weisen sie einen pH-Wert im alkalischen Bereich zwischen 8,0 und 11,5 auf, wobei die Einstellung vorzugsweise mit Ammoniak erfolgt. Es können dazu aber auch bestimmte organische Amine, z. B. Monoäthanolamin oder Triäthanolamin, Verwendung finden.

Ihre Anwendung erfolgt in bekannter Weise, indem man die Haarfärbemittel kurz vor dem Gebrauch mit einem Oxidationsmittel vermischt und das Gemisch auf das Haar aufträgt. Als Oxidationsmittel zur Entwicklung der Haarfärbung kommt hauptsächlich Wasserstoffperoxid, beispielsweise als 6%ige wäßrige Lösung, bzw. dessen Additionsverbindungen an Harnstoff, Melamin oder Natriumborat in Betracht. Die Anwendungstemperaturen der Haarfärbemittel liegen im Bereich von 15 bis 50° C.

Nach einer Einwirkungszeit von etwa 10 bis 45 Minuten, vorzugsweise etwa 30 Minuten, wird das Haar mit Wasser ausgespült und getrocknet. Gegebenenfalls wird im Anschluß an diese Spülung mit einem Shampoo gewaschen und mit einer schwachen organischen Säure, wie z. B. Zitronensäure oder Weinsäure, nachgespült.

Die erfindungsgemäßen Haarfärbemittel auf der Basis der eingangs genannten Derivate des 1,2-Methylendioxy-benzols als Kupplersubstanz führen zu Haarfärbungen mit ausgezeichneten Echtheitseigenschaften, insbesondere was die Licht-, Wasch- und Reibechtheit betrifft, und lassen sich mit Reduktionsmitteln wieder abziehen.

Hinsichtlich der färberischen Möglichkeiten bieten die erfindungsgemäßen Haarfärbemittel je nach Art und Zusammensetzung der Farbkomponenten eine breite Palette verschiedener Farbnuancen, welche sich von blonden über braune, purpurne, violette bis hin zu blauen und schwarzen Farbtönen erstreckt.

Die überlegenen färberischen Eigenschaften der Haarfärbemittel gemäß der Anmeldung werden besonders deutlich bei Verwendung von 4-Hydroxy-1,2-methylendioxy-benzol als Kupplersubstanz. Dies läßt sich insbesondere zeigen, wenn in Haarfärbemitteln auf der Basis von p-Aminophenol und Resorcin die bekannte Kupplersubstanz Resorcin durch 4-Hydroxy-1,2-methylendioxy-benzol ersetzt wird. Diese Maßnahme führt, auch gegenüber der Verwendung anderer bekannter Kupplersubstanzen, zu wesentlich farbintensiveren und natürlicheren Farbtönen. Somit ist durch den Einsatz von 4-Hydroxy-1,2-methylendioxy-benzol in den erfindungsgemäßen Haarfärbemitteln erstmals die Möglichkeit gegeben, dunkle Naturtöne auf der Basis von p-Aminophenol zu erzeugen.

Darüber hinaus liefert 4-Hydroxy-1,2-methylendioxy-benzol mit den Entwicklersubstanzen 1,4-Diaminobenzol und 2,5-Diaminotoluol sehr intensive, natürliche Farbtöne, wobei je nach Konzentration der beiden Farbvorstufen blonde bis dunkelbraune Färbungen erreicht werden. Hierbei ist von besonderer Bedeutung, daß 4-Hydroxy-1,2-methylendioxy-benzol in Kombination mit 1,4-Diaminobenzol bzw. 2,5-Diaminotoluol schon in verhältnismäßig geringen Konzentrationen als Nuanciermittel wirksam ist.

Zusammenfassend läßt sich feststellen, daß durch die Verwendung der erfindungsgemäßen Haarfärbemittel mit einem Gehalt an den genannten Derivaten des 1,2-Methylendioxybenzols als Kupplersubstanz die bestehenden Möglichkeiten zur Erzeugung von Farbnuancen auf oxidativem Wege erheblich vergrößert werden, wobei insbesondere durch die Erschließung intensiver Färbungen auf der Basis der Entwicklersubstanz p-Aminophenol nun physiologisch verträglichere Haarfärbemittel zur Verfügung stehen.

In den nachfolgenden Beispielen soll der Gegenstand der Erfindung näher erläutert werden.

## Beispiele

### Beispiel 1 Haarfärbelösung

| | |
|---|---|
| 2,0 g | 4-Hydroxy-1,2-methylendioxy-benzol |
| 2,0 g | p-Aminophenol |
| 0,8 g | Natriumhydroxid, fest |
| 0,4 g | Natriumsulfit, wasserfrei |
| 10,0 g | Laurylalkohol-diglykoläthersulfat, 28%ige wäßrige Lösung |
| 10,0 g | Isopropanol |
| 20,0 g | Ammoniak, 10%ig |
| 54,8 g | Wasser |
| 100,0 g | |

50 g des obigen Haarfärbemittels werden kurz vor dem Gebrauch mit 50 ml Wasserstoffperoxidlösung (6%ig) vermischt und das Gemisch anschließend auf blonde menschliche Haare aufgetragen. Nach einer Einwirkungszeit von 30 Minuten bei 40°C wird mit Wasser gespült und getrocknet. Das Haar hat einen natürlichen mittelbraunen Farbton mit gelblichen Reflexen erhalten.

### Beispiel 2 Haarfärbemittel in Gelform

| | |
|---|---|
| 0,6 g | 4-Hydroxy-1,2-methylendioxy-benzol |
| 0,4 g | 1,4-Diaminobenzol |
| 0,3 g | Natriumhydroxid, fest |
| 0,4 g | Natriumsulfit, wasserfrei |
| 15,0 g | Ölsäure |
| 7,0 g | Isopropanol |
| 10,0 g | Ammoniak, 22%ig |
| 66,3 g | Wasser, vollentsalzt |
| 100,0 g | |

Man vermischt kurz vor Gebrauch 50 g dieses Haarfärbemittels mit 50 ml Wasserstoffperoxidlösung (6%ig) und läßt das Gemisch 30 Minuten lang bei 40°C auf blonde menschliche Haare einwirken. Danach wird mit Wasser gespült und getrocknet. Das Haar ist in einem natürlichen Mittelbraunton gefärbt.

### Beispiel 3 Haarfärbemittel in Cremeform

| | |
|---|---|
| 0,3 g | 4-Hydroxy-1,2-methylendioxy-benzol |
| 0,4 g | p-Aminophenol |
| 0,2 g | α-Naphthol |
| 0,2 g | Natriumhydroxid, fest |
| 0,3 g | Natriumsulfit, wasserfrei |
| 3,5 g | Laurylalkohol-diglykoläthersulfat, 28%ige wäßrige Lösung |
| 15,0 g | Cetylalkohol |
| 10,0 g | Ammoniak, 22%ig |
| 70,1 g | Wasser |
| 100,0 g | |

50 g des vorstehenden Haarfärbemittels werden kurz vor dem Gebrauch mit 50 ml Wasserstoffperoxidlösung (6%ig) vermischt. Anschließend trägt man das Gemisch auf blonde menschliche Haare auf und läßt 30 Minuten lang bei 40°C einwirken. Danach wird mit Wasser gespült und getrocknet. Das Haar hat eine rötlich-mittelblonde Färbung erhalten.

Alle in der vorliegenden Anmeldung angegebenen Prozentzahlen stellen Gewichtsprozente dar.

## Patentansprüche

1. Mittel zur oxidativen Färbung von Haaren, dadurch gekennzeichnet, daß es eine Kombination von in der Haarfärbung üblichen Entwicklersubstanzen mit mindestens einem Derivat des 1,2-Methylendioxy-benzols der allgemeinen Formel

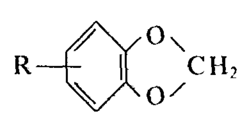

in der R eine OH-, $NH_2$-, $NHR^1$- oder $NR^1R^2$-Gruppe bedeutet, wobei $R^1$ und $R^2$ unabhängig voneinander einen Alkyl-, Hydroxyalkyl- oder Acylrest mit 1 bis 4 Kohlenstoffatomen darstellen, als Kupplersubstanz sowie gegebenenfalls zusätzlich übliche Kupplersubstanzen enthält.

2. Mittel nach Anspruch 1, dadurch gekennzeichnet, daß es als Kupplersubstanz 4-Hydroxy-1,2-methylendioxy-benzol enthält.

3. Mittel nach Anspruch 1 und 2, dadurch gekennzeichnet, daß es die erfindungsgemäßen Kupplersubstanzen in einer Konzentration von etwa 0,01 bis 3,0 Gewichtsprozent, vorzugsweise 0,1 bis 3,0 Gewichtsprozent, enthält.

4. Mittel nach Anspruch 1 bis 3, dadurch gekennzeichnet, daß es mindestens eine der Entwicklersubstanzen 1,4-Diaminobenzol, 2,5-Diaminotoluol oder p-Aminophenol enthält.

5. Mittel nach Anspruch 1 bis 4, dadurch gekennzeichnet, daß es mindestens eine der üblichen Kupplersubstanzen α-Naphthol, m-Aminophenol, m-Phenylendiamin, m-Toluylendiamin, 2,4-Diaminoanisol, 3,4-Diaminobenzoesäure und 6-Methyl-3-amino-phenol enthält.

6. Mittel nach Anspruch 1 bis 5, dadurch gekennzeichnet, daß die Gesamtmenge der enthaltenen Entwickler-Kupplersubstanz-Kombinationen 0,1 bis 5,0 Gewichtsprozent, vorzugsweise 0,5 bis 3,0 Gewichtsprozent, beträgt.

7. Mittel nach Anspruch 1 bis 6, dadurch gekennzeichnet, daß es zusätzlich mindestens einen der direktziehenden Farbstoffe Diamond Fuchsin (C.I. 42 510), Leather Ruby HF (C.I. 42 520), 2-Nitro-1,4-diaminobenzol, Acid Brown 4 (C.I. 14 805), Acid Blue 135 (C.I. 13 385), Disperse Violet 4 (C.I. 61 105), Disperse Blue 1 (C.I. 64 500), Disperse Red 15 (C.I. 60 710), Disperse Violet 1 (C.I. 61 100), 1,4,5,8-Tetraamino-anthrachinon und 1,4-Diamino-anthrachinon enthält.

8. Mittel nach Anspruch 1 bis 7, dadurch gekennzeichnet, daß es zusätzlich Antioxidantien, vorzugsweise Ascorbinsäure oder Natriumsulfit, enthält.

9. Mittel nach Anspruch 1 bis 8, dadurch gekennzeichnet, daß es zusätzlich Netzmittel, Emulgatoren und/oder Verdicker enthält.

## Claims

1. Medium for the oxidative colouring of hair, characterised in that it contains a combination of conventional hair colouring development substances with at least one 1,2-methylendioxy-benzene derivative as coupler substance of the general formula:

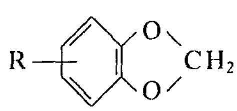

in which R represents an OH-, $NH_2$-, $NHR^1$- or $NR^1R^2$ group, in which $R^1$ and $R^2$ independently represent an alkyl, hydroxyalkyl, or acyl residue with 1 to 4 carbon atoms, and also optionally additional conventional coupler substances.

2. Medium according to claim 1, characterised in that it contains, as coupler substance, 4-hydroxy-1,2-methylendioxy-benzene.

3. Medium according to claim 1 and 2, characterised in that it contains, as the coupler substance according to the invention in a concentration from about 0.01 to 3.0 wt. %, preferably 0.1 to 3.0 wt. %.

4. Medium according to claim 1 to 3, characterised in that it contains at least one of the developer substances 1,4-diaminobenzene, 2,5-diaminotoluene or p-aminophenol.

5. Medium according to claim 1 to 4, characterised in that it contains at least one of the conventional coupler substances α-naphthol, m-aminophenol, m-phenylendiamine, m-tolylendiamine, 2,4-diaminoanisole, 3,4-diaminobenzoic acid, and 6-methyl-3-amino-phenol.

6. Medium according to claim 1 to 5, characterised in that the total amount of the developer-coupler substances combination present, amounts to 0.1 to 5.0 wt. %, preferably 0.5 to 3.0 wt. %.

7. Medium according to claim 1 to 6, characterised in that it additionally contains at least one of the direct dyes Diamond Fuchsin (C.I. 42 510), Leather Ruby HF (C.I. 42 520), 2-nitro-1,4-diaminobenzene, Acid Brown 4 (C.I. 14 805), Acid Blue 135 (C.I. 13 385), Disperse Violet 4 (C.I. 61 105), Disperse Blue 1 (C.I. 64 500), Disperse Red 15 (C.I. 60 710), Disperse Violet 1 (C.I. 61 100), 1,4,5,8-tetraamino-anthraquinone and 1,4-diamino-anthraquinone.

8. Medium according to claim 1 to 7, characterised in that it contains additional antioxidants, preferably ascorbic acid or sodium sulphite.

9. Medium according to claim 1 to 8, characterised in that it contains additional wetting agents, emulsifiers and/or thickeners.

## Revendications

1. Préparation pour la teinture oxydante des cheveux, caractérisée en ce qu'elle contient une combinaison d'agents de développement usuels pour la coloration de cheveux, avec comme agents de copulation ou de condensation un ou plusieurs dérivés du 1,2-méthylène-dioxybenzène de furmule générale

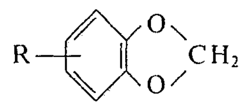

où R représente un groupe OH-, $NH_2$-, $NHR^1$-, ou $NR^1R^2$-, les radicaux $R^1$ et $R^2$ figurant, indépendamment l'un de l'autre, un groupe alkyle, hydroxy-alkyle ou acyle ayant de 1 à 4 atomes de carbone, ladite préparation contenant le cas échéant en outre, d'autres agents de condensation usuels.

2. Préparation selon la revendication 1, caractérisée en ce qu'elle contient, en tant qu'agent de condensation, le 4-hydroxy-1,2-méthylène-dioxy-benzène.

3. Préparation selon les revendications 1 et 2, caractérisée en ce qu'elle contient les agents de condensation, selon l'invention, à une concentration approximative de 0,01% à 3,0% en poids, ladite concentration étant comprise de préférence entre 0,1% et 3,0%.

4. Préparation selon les revendications 1 à 3, caractérisée en ce qu'elle contient au moins un des agents de développement 1,4-diaminobenzène, 2,5-diaminotoluène ou p-aminophénol.

5. Préparation selon les revendications 1 à 4, caractérisée en ce qu'elle contient au moins un des agents de condensation usuels tels que le α-naphtol, le m-aminophénol, le m-phénylènediamine, le m-tolylène-diamine, le 2,4-diaminoanisol, l'acide 3,4-diaminobenzoique et le 6-méthyl-3-aminophénol.

6. Préparation selon les revendications 1 à 5, caractérisée en ce que la quantité totale des combinaisons d'agents de développement et de condensation varie de 0,1% à 5,0% en poids, la proportion préférentielle variant entre 0,5% et 3,0%.

7. Préparation selon les revendications 1 à 6, caractérisée en ce qu'elle contient en outre l'un ou plusieurs des colorants à prise directe tels que ceux ci-après dénommés: »Diamond Fuchsin« (C. I. 42 510), »Leather Ruby« HF (C. I. 42 520), le 2-nitro-1,4-diaminobenzène, »Acid Brown 4« (C. I. 14 805), »Acid Blue 135« (C. I. 13 385), »Disperse Violet 4« (C. I. 61 105), »Disperse Blue 1« (C. I. 64 500), »Disperse Red 15« (C. I. 60 710), »Disperse Violet 1« (C. I. 61 100),

le 1,4,5,8-tetraamino-anthraquinone et le 1,4-diamino-anthraquinone.

8. Préparation selon les revendications 1 à 7, caractérisée en ce qu'elle contient en outre des antioxydants, de préférence l'acide ascorbique ou le sulfite de sodium.

9. Préparation selon les revendications 1 à 8, caractérisée en ce qu'elle contient en outre des mouillants, des émulsifiants et/ou des épaississants.